# EUROPEAN PATENT SPECIFICATION

(11) **EP 2 172 902 B1**
(45) Date of publication and mention of the grant of the patent: **16.11.2011**
(21) Application number: 09012427.2
(22) Date of filing: 30.09.2009
(51) Int. Cl.: G06T 11/20, A61B 1/00, G06F 17/30

(54) **Image display apparatus, image display method, and image display program**
Bildanzeigevorrichtung, Bildanzeigeverfahren und Bildanzeigeprogramm
Appareil d'affichage d'images, procédé d'affichage d'images et programme d'affichage d'images

(30) Priority: 30.09.2008 JP 2008255348
(43) Date of publication of application: 07.04.2010
(73) Proprietor: Olympus Medical Systems Corp., Tokyo 151-0072 (JP)
(72) Inventor: Zui, Eri, Tokyo 151-0072 (JP); Taniguchi, Katsuyoshi, Tokyo 151-0072 (JP)
(74) Representative: von Hellfeld, Axel

(56) References cited:
- EP-A1- 1 787 574
- EP-A1- 1 883 020
- EP-A1- 1 922 977
- EP-A1- 1 922 984
- HYUNMO KANG ET AL: "Visualization methods for personal photo collections: browsing and searching in the PhotoFinder" MULTIMEDIA AND EXPO, 2000. ICME 2000. 2000 IEEE INTERNATIONAL CONFEREN CE ON NEW YORK, NY, USA 30 JULY-2 AUG. 2000, PISCATAWAY, NJ, USA,IEEE, US LNKD- DOI:10.1109/ICME.2000.871061, vol. 3, 30 July 2000 (2000-07-30), pages 1539-1542, XP010512799 ISBN: 978-0-7803-6536-0

## Description

### TECHNICAL FIELD

The present invention relates to an image display apparatus that displays a series of images obtained by capturing, in time series, the inside of organs of a subject, e.g., a patient. In particular, the present invention relates to an image display apparatus, an image display method and an image display program that can display a list of images in the series of images.

### BACKGROUND ART

Conventionally, in the field of endoscope, a swallowable capsule endoscope that has imaging and radio communication functions in its capsule-type casing is well known. Such a capsule endoscope is introduced into organs of a subject, e.g., a patient, orally or by other means. Then, while moving through organs due to peristalsis or the like, the capsule endoscope inside the subject captures images inside the organs of the subject (hereinafter, "in-vivo images") and wirelessly transmits the obtained in-vivo images to the outside. Until the capsule endoscope, which has been introduced into the subject, is excreted by the subject, the capsule endoscope sequentially captures in-vivo images in time series and wirelessly transmits the obtained in-vivo images in time series to the outside of the subject.

Each in-vivo image wirelessly transmitted in time series by the capsule endoscope is received by a receiving apparatus that is located outside the subject. The receiving apparatus stores the in-vivo image group received in time series from the capsule endoscope in a storage medium, which is previously inserted into the receiving apparatus. Then, the storage medium storing the in-vivo image group is detached from the receiving apparatus and inserted into the image display apparatus. The image display apparatus extracts the in-vivo image group stored in the inserted storage medium and sequentially displays each of the obtained in-vivo images on a display. Each of the in-vivo images displayed sequentially by the image display apparatus is observed by medical workers, such as a doctor and a nurse. By observing the in-vivo images, the inside of organs of the subject can be observed (examined).

As an example of such image display apparatuses that display in-vivo image groups of a subject, as described above, there is known an image display apparatus that divides an image stream captured by the capsule endoscope into a plurality of image streams and then displays the image streams on a display substantially at the same time (Japanese Patent Application Laid-Open No. 2006-305369). Furthermore, there is known an image display apparatus that classifies a plurality of images based on relation information among image data and displays a list of classified images (Japanese Patent Application Laid-Open No. 2004-13575).

When the conventional image display apparatus described above displays a list of in-vivo images, the image display apparatus extracts the in-vivo images to be displayed from an in-vivo image group of a subject based on an extraction condition previously set for images to be displayed and displays a list of the extracted in-vivo images on a screen. When the conventional image display apparatus later changes a level of a threshold value or the like in the extraction condition for the images to be displayed, the image display apparatus re-extracts in-vivo images to be displayed based on the post-change extraction condition and displays a list of the re-extracted in-vivo images. The list of the in-vivo images displayed on a screen can include both the in-vivo images that have been extracted based on the previous extraction condition and the in-vivo images that are newly extracted based on the post-change extraction condition.

The problem is that it is difficult, using the conventional image display apparatus, to visually distinguish the in-vivo images that have been extracted based on the previous extraction condition from the in-vivo images that are newly extracted based on the post-change extraction condition in a list of in-vivo images displayed on a screen. Accordingly, when observing a list of in-vivo images, users can end up observing again the in-vivo images that have been already observed. As a result, observing the in-vivo images of the subject takes users a significant amount of time and burden.

Document EP 1 787 574 A1 concerns an image display apparatus including a display unit that sequentially displays a plurality of stored images in an imaging order thereof, and a control unit that causes the display unit to display an extracted image preliminarily extracted from the plurality of stored images under a predetermined extraction condition.

The present invention is made in view of the above. An object of the present invention is to provide an image display apparatus, an image display method, and an image display program that can reduce burden on users when observing each in-vivo image in a list displayed on a screen and thus allow users to efficiently observe the in-vivo images in a short time.

### DISCLOSURE OF INVENTION

Some or all of the above problems are overcome by an image display apparatus having the features of claim 1, an image display method having the features of claim 7, and an image display program having the features of claim 13.

An image display apparatus according to an aspect of the present invention includes an image obtaining unit that obtains a series of images captured in time series; an extraction-condition setting unit that sets an image-extraction condition that can be changed in a hierarchical manner; an image extracting unit that extracts images from a series of images based on the image-extraction condition set by the extraction-condition setting unit; a display unit that displays a list of images extracted by the image extracting unit; and a display controller that controls the display unit so that when the image-extraction condition is changed in a hierarchical manner by the extraction-condition setting unit, new images that are newly extracted according to the post-change image-extraction condition, out of images extracted according to the image-extraction condition that is changed by the image extracting unit are displayed in a different display pattern from that of old images that have been extracted according to the pre-change image-extraction condition.

An image display method according to another aspect of the present invention includes changing, in a hierarchical manner, an image-extraction condition for extracting in-vivo images to be displayed in a list from the a series of images captured in time series; re-extracting in-vivo images to be displayed in a list from the series of images according the post-change image-extraction condition changed at the changing; and displaying on a display unit a list of in-vivo images re-extracted at the extracting so that new images that are newly extracted according to the post-change image-extraction condition are displayed in a different display pattern from that of old images that are extracted according to the pre-change image-extraction condition.

An image display program according to still another aspect of the present invention causes a computer to execute the image display method according to the present invention.

The above and other features, advantages and technical and industrial significance of this invention will be better understood by reading the following detailed description of presently preferred embodiments of the invention, when considered in connection with the accompanying drawings.

### BRIEF DESCRIPTION OF DRAWINGS

FIG. 1 is a schematic diagram of an exemplary configuration of an in-vivo information obtaining system including an image display apparatus in accordance with a first embodiment of the present invention.
FIG. 2 is a schematic block diagram of an exemplary configuration of an image display apparatus in accordance with a first embodiment of the present invention.
FIG. 3 is a flowchart illustrating a procedure of an image display apparatus for displaying a list of in-vivo images.
FIG. 4 is a schematic diagram illustrating operations of an image display apparatus for extracting in-vivo images to be displayed in a list on an image-extraction condition that can be changed in a hierarchical manner.
FIG. 5 is a schematic diagram of a display pattern for a list of in-vivo images displayed by an image display apparatus in accordance with the first embodiment of the present invention.
FIG. 6 is a schematic block diagram of an exemplary configuration of an image display apparatus in accordance with a second embodiment of the present invention.
FIG. 7 is a schematic diagram illustrating a display pattern for a list of in-vivo images displayed by an images display apparatus in accordance with the second embodiment of the present invention.
FIG. 8 is a schematic block diagram of an exemplary configuration of an image display apparatus in accordance with a third embodiment of the present invention.
FIG. 9 is a schematic diagram of a display pattern for a list of in-vivo images displayed by an image display apparatus in accordance with the third embodiment of the present invention.
FIG. 10 is a schematic diagram of an exemplary configuration of an image display apparatus in accordance with a fourth embodiment of the present invention.
FIG. 11 is a schematic diagram illustrating a display pattern for displaying a list of in-vivo images with level information on an image-extraction condition used by an image display apparatus in accordance with the fourth embodiment of the present invention.
FIG. 12 is a schematic diagram illustrating a variation of level information on an image-extraction condition that is displayed together with in-vivo images to be displayed in a list.
FIG. 13 is a schematic diagram illustrating another variation of level information on an image-extraction condition that is displayed together with in-vivo images to be displayed in a list.

### BEST MODE(S) FOR CARRYING OUT THE INVENTION

Exemplary embodiments of an image display apparatus, an image display method, and an image display program in accordance with the present invention are described with reference to the accompanying drawings. The embodiments do not limit the scope of the present invention.

### First Embodiment

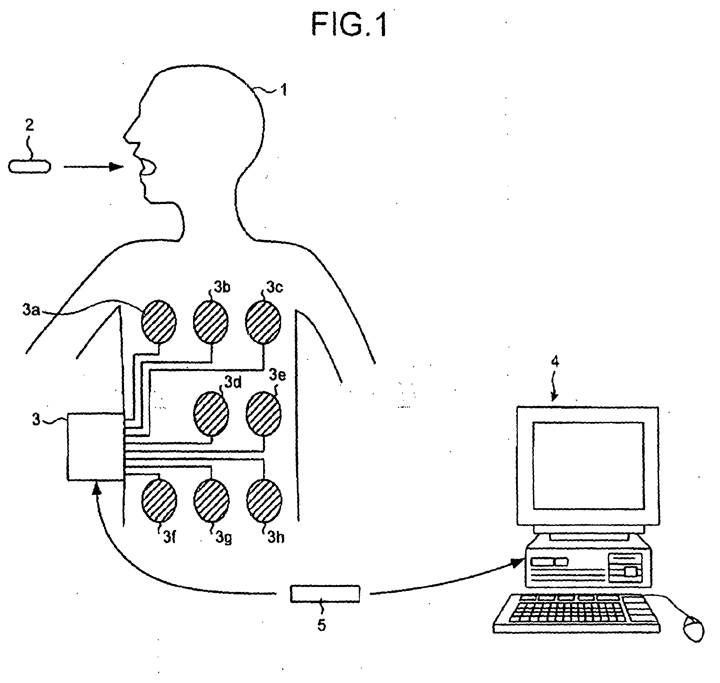

FIG. 1 is a schematic diagram of an in-vivo information obtaining system including an image display apparatus in accordance with a first embodiment of the present invention. As shown in FIG. 1, the in-vivo information obtaining system in accordance with the first embodiment includes a capsule endoscope 2 that captures in-vivo images of a subject 1, a receiving apparatus 3 that receives image signals wirelessly transmitted from the capsule endoscope 2, an image display apparatus 4 that displays the in-vivo images captured by the capsule endoscope 2, and a portable storage medium 5 that is used for transferring data between the receiving apparatus 3 and the image display apparatus 4.

The capsule endoscope 2 is a capsule endoscope apparatus of a size that can be introduced into organs of the subject 1. The capsule endoscope 2 has imaging and radio communication functions in its capsule-type casing. In detail, the capsule endoscope 2 is introduced into organs of the subject 1, orally or by other means. Then, while moving through the organs due to peristalsis or the like, the capsule endoscope 2 inside the subject 1 sequentially captures in-vivo images in time series and wirelessly transmits image signals of the obtained in-vivo images sequentially to the receiving apparatus that is located outside. Until the capsule endoscope 2, which has been introduced into the organs of the subject 1, is excreted by the subject 1, the capsule endoscope 2 keeps performing capturing operations and wireless-transmitting operations of the in-vivo images sequentially.

The receiving apparatus 3 includes a plurality of receiving antennas 3a to 3h and receives, via at least one of the receiving antennas 3a to 3h, radio-communication signals from the capsule endoscope 2 inside the subject 1. The receiving apparatus 3 performs predetermined transmission processes such as a demodulation process on the radio-communication signals received from the capsule endoscope 2, extracts image signals from the radio-communication signals, and generates in-vivo images of the subject 1 based on the extracted image signals. The receiving apparatus 3 keeps performing these processes in order to obtain an in-vivo image group of the subject 1 captured by the capsule endoscope 2. Furthermore, the storage medium 5 is previously inserted into the receiving apparatus 3. The receiving apparatus 3 stores therein image data in an in-vivo image group of the subject 1 in association with time data such as capture time of each in-vivo image.

Furthermore, the receiving antennas 3a to 3h of the receiving apparatus 3 may be located spreading over the body surface of the subject as shown in FIG. 1 or may be located on a jacket to be worn by the subject 1. Furthermore, the number of receiving antennas included in the receiving apparatus 3 is not limited to eight and may be one or more.

The image display apparatus 4 has a configuration like that of a workstation and obtains various types of data such as the in-vivo image group of the subject 1 and displays these obtained data. In detail, the storage medium 5, which is detached from the receiving apparatus 3 described above, is attached to the image display apparatus 4, and thus the data stored in the storage medium 5 is transferred to the image display apparatus 4. As described, the image display apparatus 4 obtains various types of data such as the in-vivo image group of the subject 1 captured by the capsule endoscope 2. The image display apparatus 4 displays each in-vivo image of the obtained in-vivo image group as a still or moving image or displays a list of invivo images extracted from the in-vivo image group. Each in-vivo image displayed by the image display apparatus 4 is observed by medical workers, such as doctors and nurses. This observation of each in-vivo image allows medical workers to observe the inside of organs of the subject 1 so that the subject 1 can be diagnosed.

The storage medium 5 is a portable storage medium that is used for transferring data between the receiving apparatus 3 and the image display apparatus 4 described above. In detail, the storage medium 5 can be detached from and attached to both the receiving apparatus 3 and the image display apparatus 4. When inserted into the receiving apparatus 3 or the image display apparatus 4, the storage medium 5 enables data output or data storage. When inserted into the receiving apparatus 3, the storage medium 5 stores therein various types of data such as the in-vivo image group that is received by the receiving apparatus 3 from the capsule endoscope 2. After the storage medium 5 storing various types of data is detached from the receiving apparatus 3, the storage medium 5 is inserted into the image display apparatus 4. Then, the data stored in the storage medium 5 is transferred to the image display apparatus 4.

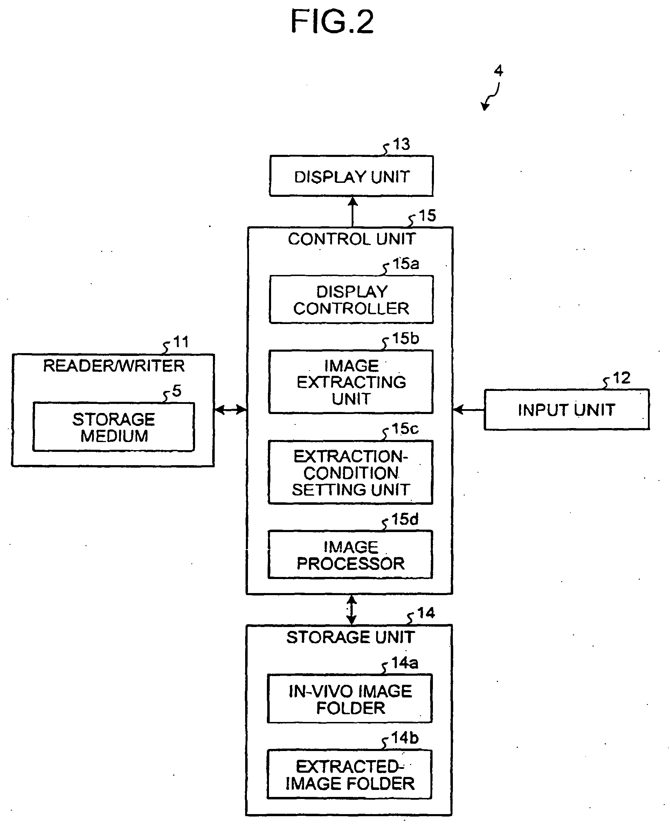

A configuration of the image display apparatus in accordance with the first embodiment of the present invention is described in detail. FIG. 2 is a schematic block diagram of an exemplary configuration of an image display apparatus in accordance with the first embodiment of the present invention. As shown in FIG. 2, the image display apparatus 4 in accordance with the first embodiment includes a reader/writer 11 that loads the data stored in the storage medium 5, an input unit 12 that inputs various types of information, a display unlit 13 that displays in-vivo images or the like of the subject 12, a storage unit 14 that stores therein various types of data loaded by the reader/writer 11 or other data, and a control unit 15 that controls each of the components of the image display apparatus 4.

The reader/writer 11 functions as an image obtaining unit that obtains the in-vivo image group of the subject 1 captured by the capsule endoscope 2 described above. In detail, the storage medium 5, which is detached from the receiving apparatus 3 described above, is inserted into the reader/writer 11 in a manner such that the storage medium 5 can be attached to or detached from the reader/writer 11. Then, the reader/writer 11 loads the data stored in the storage medium 5 under the control of the control unit 15. As described, the reader/writer 11 obtains various types of data such as the in-vivo image group of the subject 1 and transfers various types of obtained data to the control unit 15. The in-vivo image group obtained by the reader/writer 11 is a series of images obtained by the capsule endoscope 2, described above, capturing in time series the inside of the organs of the subject 1. When the storage medium 5, which is initialized, is inserted into the storage medium 5 instead, the reader/writer 11 writes data corresponding to writing instructions from the control unit 15 into the storage medium 5. In this case, the reader/writer 11 writes specific data such as patient name, patient ID, examination date, and examination ID of the subject 1 into the storage medium 5.

The input unit 12 can be an input device such as a keyboard and a mouse. The input unit 12 is used for inputting various types of information into the control unit 15 according to inputting operations by users. Various types of information input from the input unlit 12 to the control unit 15 are, for example, pierces of information such as various types of instruction information for instructing the control unit 15, specific data on the subject 1, and an image-extraction condition for extracting in-vivo images to be displayed in a list.

The display unit 13 can be a display such as a CRT display and a liquid crystal display, which can display images. The display unit 13 displays various types of information corresponding to displaying instructions from the control unit 15. In detail, the display unit 13 displays the in-vivo image group of the subject captured by the capsule endoscope 2 described above, the specific data of the subject 1, input information from the input unit 12, various types of GUI (Graphical User Interface) for inputting operations and displaying operations, and the like. Under the control of the control unit 15, the display unit 13 displays each in-vivo image in the in-vivo image group of the subject 1 as a still image or a moving image in time series or inverted time series or displays a list of in-vivo images extracted from the in-vivo image group of the subject 1.

The storage unit 14 can be a nonvolatile storage medium such as an EEPROM and a hard disk, which stores data in a rewritable manner. The storage unit 14 stores therein various types of data according to writing instructions from the control unit 15 and transmits stored data, according to reading instructions from the control unit 15, to the control unit 15. In detail, under the control of the control unit 15, the storage unit 14 stores therein information input from the input unit 12 such as the specific data of the subject 1, data obtained by the reader/writer 11 such as the in-vivo image group of the subject 1, various types of data such as the image-extraction condition for extracting in-vivo images to be displayed in a list, and various types of information. Furthermore, the storage unit 14 includes an in-vivo image folder 14a that is a storage area for storing all in-vivo images obtained, and an extracted-image folder 14b that is a storage area for storing in-vivo images extracted from the in-vivo image group in the in-vivo image folder 14a. The storage unit 14 stores the in-vivo image group of the subject described above in the in-vivo image folder 14a and stores, the in-vivo images extracted from the in-vivo image group in the extracted-image folder 14b.

The control unit 15 may include a storage unit for storing programs corresponding to functions of the image display apparatus 4 and a CPU for executing programs in the storage. The control unit 15 controls operations of the components of the image display apparatus 4, i.e., the reader/writer 11, the input unit 12, the display unit 13, and the storage unit 14 and controls the input and output of signals among these components. In detail, based on instruction information input from the input unit 12, the control unit 15 controls the reader/writer 11 as the reader/writer 11 loads the data stored in the storage medium 5 and controls the storage unit 14 as the storage unit 14 stored therein the loaded stored data. Furthermore, the control unit 15 controls the reader/writer 11 as the reader/writer 11 stores the specific data of the subject 1 input from the input unit 12 in the storage medium 5.

Furthermore, the control unit 15 includes a display controller 15a that controls display operations of the display unit 13, an image extracting unit 15b that extracts in-vivo images to be displayed in a list, an extraction-condition setting unit 15c that sets an image-extraction condition that can be changed in a hierarchical manner, and an image processor 15d that performs various types of processes on each in-vivo image in the in-vivo image group.

Based on the instruction information input from the input unit 12, the display controller 15a controls the display unit 13 as the display unit 13 displays each in-vivo image in the in-vivo image group of the subject 1 as a still or moving image. In this case, the display controller 15a reads the in-vivo image group of the subject 1 from the in-vivo image folder 14a in the storage unit 14. Then, the display controller 15a controls the display unit 13 as the display unit 13 displays each in-vivo image in the read in-vivo image group in time series or inverted time series as a still or moving image. Based on the input information from the input unit 12, the display controller 15a previously sets the number of in-vivo images to be displayed on a screen at one time. Based on the instruction information from the input unit 12, the display controller 15a controls the display unit 13 as the display unit 13 displays a list of in-vivo images in the in-vivo image group of the subject 1. In this case, the display controller 15a reads in-vivo images from the extracted-image folder 14b in the storage unit 14 and controls the display unit 13 as the display unit 13 displays a list of the in-vivo images according to the number of displayed images that is previously set. The in-vivo images stored in the extracted-image folder 14b are extracted by the image extracting unit 15b from the in-vivo image group of the subject 1.

When the extraction-condition setting unit 15c changes the image-extraction condition in a hierarchical manner, the display controller 15a displays a list of in-vivo images on the display unit 13 in a manner such that in-vivo images that are newly displayed according to the post-change image-extraction condition are displayed in a different display pattern from that of in-vivo images that have been displayed according to the pre-change image-extraction condition. In this case, the display controller 15a controls the display unit 13 as the display unit 13 displays newly extracted images in a different display pattern from that of the old in-vivo images in the in-vivo images extracted by the image extracting unit 15b according to the changed image-extraction condition (i.e., in-vivo images to be displayed in a list). In detail, the display controller 15a controls the display unit. 13 as the display unit 13 highlights the in-vivo images that are newly extracted according to the changed image-extraction condition in the in-vivo images to be displayed in a list. As described, the display controller 15a ensures that the new in-vivo images and old in-vivo images are displayed in different display patterns.

The new in-vivo images described above are the in-vivo images that are newly extracted by the image extracting unit 15b according to the changed image-extraction condition. Furthermore, the old in-vivo images described above are the in-vivo images that have been extracted by the image extracting unit 15b according to the pre-change image-extraction condition.

The image extracting unit 15b functions as an image extractor that extracts in-vivo images to be displayed in a list by the display unit 13. In detail, the image extracting unit 15b extracts in-vivo images from the in-vivo image group of the subject 1 in the in-vivo image folder 14a according to the image-extraction condition that is set by the extraction-condition setting unit 15c. In this case, the image extracting unit 15b extracts the in-vivo images corresponding to the image-extraction condition from the in-vivo image group of the subject 1. The in-vivo images extracted by the image extracting unit 15b are stored in the extracted-image folder 14b in the storage unit 14. Every time the extraction-condition setting unit 15c changes the image-extraction condition in a hierarchical manner, the image extracting unit 15b newly extracts the in-vivo images corresponding to the image-extraction condition, in the in-vivo image group of the subject 1 from the in-vivo image group of the subject 1.

The extraction-condition setting unit 15c sets the image-extraction condition for the image extracting unit 15b to use for extracting in-vivo images to be displayed in a list from the in-vivo image group of the subject 1. Furthermore, the extraction-condition setting unit 15c changes a level of the image-extraction condition in a hierarchical manner based on input information from the input unit 12. That is, the extraction-condition setting unit 15c can set the image-extraction condition at multi levels based on the input information from the input unit 12. The image-extraction condition is stored in the storage unit 14 under the control of the control unit 15 and is read by the control unit 15 as needed. The image-extraction condition set by the extraction-condition setting unit 15c can be changed in a hierarchical manner. For example, the image-extraction conditions can be a threshold value for the similarity among in-vivo images that are successive in time series, a threshold value indicating a value related to information that bleeding regions exist in the in-vivo images as photographic subjects, and a threshold value indicating a value related to results of image processing on in-vivo images captured with special light.

The image processor 15d performs predetermined image processes on each in-vivo image in the in-vivo image group of the subject 1 and calculates information required for extracting processes for in-vivo images that are performed by the image extracting unit 15b described above. In detail, the image processor 15d reads the in-vivo image group of the subject 1 stored in the in-vivo image folder 14a in the storage unit 14 and extracts color information of each in-vivo image in the in-vivo image group. Based on the color information of each in-vivo image, the image processor 15d calculates similarity among in-vivo images that are successive in time series in the in-vivo image group for each image. The image processor 15d may calculate a motion vector among adjacent images in the in-vivo image group so that the image processor 15d, based on the calculated motion vector of each in-vivo image, can calculate similarity among in-vivo images for each image. The image processor 15d associates each similarity calculated for each image with each in-vivo image in the in-vivo image group. The control unit 15 stores each similarity in the storage unit 14 in association with each in-vivo image. The similarity of each in-vivo image stored in the storage unit 14 is read by the control unit 15 as needed and is used for the image extracting unit 15b described above to extract in-vivo images to be displayed in a list.

The control unit 15 may include a storage unit that stores, when the image-extraction condition is changed, the pre-change extraction condition and the post-change image-extraction condition. The control unit 15 can store the image-extraction condition that is set in a hierarchical manner or at multi levels. As a result, the control unit 15 can perform the same image-extraction process again.

The image extracting unit 15b may include an extraction-result manager that manages information indicating, for each in-vivo image extracted based on the pre-change image-extraction condition, that in-vivo images have been extracted for each in-vivo image. Therefore, the image extracting unit 15b can compare the extraction results of the in-vivo images that are extracted based on the image-extraction conditions set in a hierarchical manner or at multi levels, and the display controller 15a can display these extraction results on the display unit 13 in different patterns.

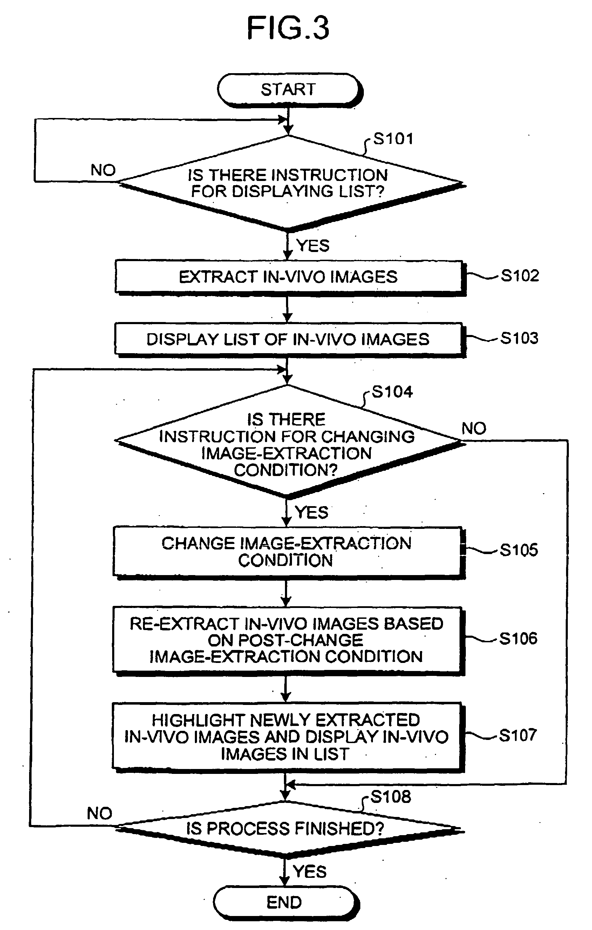

Operations of the image display apparatus 4 in accordance with the first embodiment of the present invention are described. FIG. 3 is a flowchart illustrating a procedure for displaying a list of in-vivo images of an image display apparatus. The image display apparatus 4 in accordance with the first embodiment extracts in-vivo images to be displayed in a list from the obtained in-vivo image group of the subject 1 and displays these in-vivo images by a predetermined number of images at a time. In this case, the image display apparatus 4 displays new in-vivo images in a different display pattern from that of old in-vivo images in the extracted is-vivo images.

In detail, as shown in FIG. 3, the control unit 15 of the image display apparatus 4 in accordance with the first embodiment determines first whether there is an instruction for displaying a list of in-vivo images (Step S101). When the instruction information for displaying a list is input from the input unit 12 at Step 5101, the control unit 15 determines that there is an instruction for displaying a list of in-vivo images. When the instruction information for displaying a list is not input, the control unit 15 determines that there is no instruction for displaying a list of in-vivo images. When the control unit 15 determines that there is no instruction for displaying a list of in-vivo images (Step S101, No), the control unit 15 repeats Step S101 until the instruction information for displaying a list is input from the input unit 12.

When the control unit 15 determines that there is an instruction for displaying a list of in-vivo images at Step S101 (Step S101, Yes), the control unit 15 extracts in-vivo images to be displaying in a list from the in-vivo image group obtained previously (Step S102).

At Step S102, the image extracting unit 15b extracts in-vivo images matching the image-extraction condition that is previously set by the extraction-condition setting unit 15c from the in-vivo image group of the subject 1 stored in the in-vivo image folder 14a of the storage unit 14. In this case, the image extracting unit 15b compares the similarity of each in-vivo image in the in-vivo image group with the image-extraction condition, and, based on a result of the comparison, the image extracting unit 15b extracts in-vivo images. As described above, the similarity of each in-vivo image is previously calculated by the image processor 15d. The control unit 15 controls the storage unit 14 as the storage unit 14 stores the extracted in-vivo images in the extracted-image folder 14b.

Then, the control unit 15 controls the display unit 13 as the display unit 13 displays a list of in-vivo images extracted at Step S102 (Step S103). In this case, the display controller 15a reads, from the extracted-image folder 14b of the storage unit 14, the in-vivo images. extracted by the image extracting unit 15b described above. The display controller 15a controls the display unit 13 as the display unit 13 displays a list of in-vivo images at a time by the predetermined number of images to be displayed on a screen.

With a list of the in-vivo images being displayed, the control unit 15 determines whether there is an instruction for changing the image-extraction condition for in-vivo images to be displayed in a list (Step S104) When the instruction information for changing the image-extraction condition is input from the input unit 12 at Step S104, the control unit 15 determines that there is an instruction for changing the image-extraction condition. When the instruction information for changing the image-extraction condition is not input, the control unit 15 determines that there is no instruction for changing the image-extraction condition.

When the control unit 15 determines that there is an instruction for changing the image-extraction condition (Step S104, Yes), the control unit 15 changes the image-extraction condition for in-vivo images to be displayed in a list (Step S105). Based on the instruction information for changing the image-extraction condition that is input from the input unit 12, the extraction-condition setting unit 15c changes the image-extraction condition for in-vivo images to be displayed in a list, in a hierarchical manner. In this case, the extraction condition setting unit 15c increases or decreases the level of the image-extraction condition (e.g., threshold value for the similarity of in-vivo image) based on the instruction information, for changing the image-extraction condition.

The control unit 15 re-extracts in-vivo images to be displayed in a list based on the post-change image-extraction condition set by the extraction-condition setting unit l5c at Step S105 described above (Step S106). At Step S106, the image extracting unit 15b re-extiacts in-vivo images matching the post-change image-extraction condition from the in-vivo image group of the subject in the in-vivo image-folder 14a. In this case, the image extracting unit 15b re-compares the similarity of each in-vivo image in the in-vivo image group with the image-extraction condition, and based on a result of the recomparison, the image extracting unit 15b re-extracts the in-vivo images. The control unit 15 controls the storage unit 14 as the storage unit 14 overwrites the re-extracted in-vivo images into the extracted-image folder 14b. As a result, the in-vivo images to be displayed in a list are updated by the re-extracted in-vivo images.

Then, the control unit 15 highlights the in-vivo images that are newly extracted from the in-vivo image group of the subject 1 after the image-extraction condition is changed at Step S106 and makes the display unit 13 display in-vivo images (Step S107). At Step S107, the display controller 15a controls the display unit 13 as the display unit 13 displays a list of in-vivo images by the predetermined number of images to be displayed at a time, the in-vivo images being re-extracted by the image extracting unit 15b based on the post-change image-extraction condition as described above. At the same time, the display.controller 15a controls the display unit 13 as the display unit 13 highlights in-vivo images that are not extracted on the pre-change image-extraction condition but newly extracted on the post-change image-extraction condition in the re-extracted in-vivo images. In this case, the display controller 15a determines which in-vivo images are new ones based on a range of the image-extraction condition between the pre-change image extraction condition and the post-change image-extraction condition.

Under the control of the display controller 15a, the display unit 13 displays the old in-vivo images that have been extracted on the post-change image-extraction condition and highlights the new in-vivo images that are newly extracted on the post-change image-extraction condition in a different display pattern from that of the old in-vivo images. In this manner, the in-vivo images are displayed in a list.

Then, the control unit 15 determines whether the process for displaying a list of in-vivo images is to be terminated (Step S108). When instruction information for terminating the process is input from the input unit 12, the control unit 15 determines that the process is to be terminated based on the input instruction information (Step S108, Yes) and terminates the process. When instruction information for terminating the process is not input at Step S108, the control unit 15 determines that the process is not to be terminated (Step S108, No) and returns to Step S104 described above to repeat the procedure following Step S104.

When the control unit 15 determines that there is no instruction for changing the image-extraction condition the control unit 15 at Step S104 described above (Step S104, No), the control unit 15 proceeds to Step S108 and repeats the procedure following Step S108.

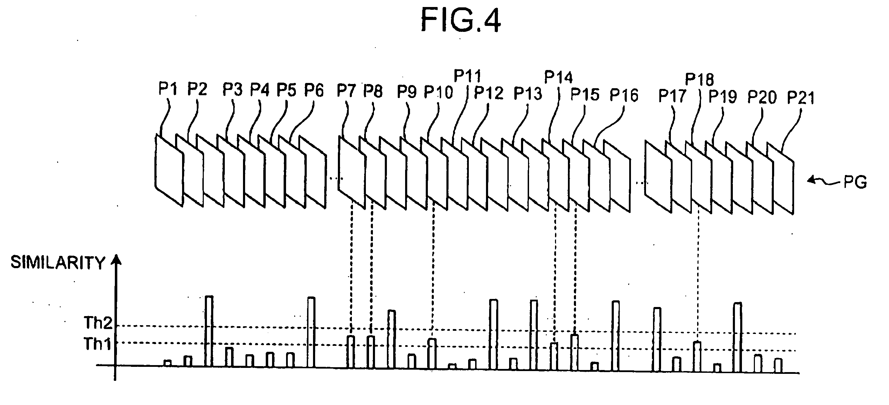

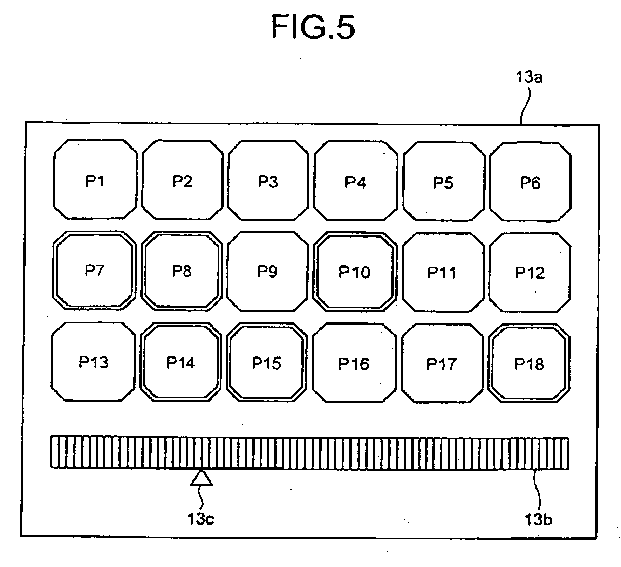

Operations for displaying a list of in-vivo images of the image display apparatus 4 are described in detail referring to an example operation of hierarchically changing the threshold value for the similarity of in-vivo images that is used as the image-extraction condition for in-vivo images. FIG. 4 is a schematic diagram illustrating operations of an image display apparatus for extracting in-vivo images to be displayed in a list on the image-extraction condition that can be changed in a hierarchical manner. FIG. 5 is a schematic diagram of a display pattern for a list of in-vivo images displayed by an image display apparatus in accordance with the first embodiment of the present invention.

In preparations for the image display apparatus 4 in accordance with the first embodiment to display a list of in-vivo images, the display controller 15a previously sets the number of in-vivo images to be displayed on a screen at once based on input information from the input unit 12. The extraction-condition setting unit 15c sets a threshold value Th1 for the similarity of in-vivo images as an initial value of the image-extraction condition that can be changed in a hierarchical manner. The threshold value Th1 used as the image-extraction condition may be a default value that is previously set on the extraction-condition setting unit 15c or may be a value that is newly set by the extraction-condition setting unit 15c based on input information from the input unit 12.

When instruction information for displaying a list of in-vivo images is input from the input unit 12 to the image display apparatus 4, the control unit 15 determines, based on the instruction information for displaying a list that is input from the input unit 12, that there is an instruction for displaying in-vivo images. In this case, the image extracting unit 15b extracts in-vivo images matching the image-extraction condition set by the image-extraction condition setting unit 15c from an in-vivo image group PG of the subject 1 that is stored in the in-vivo image folder 14a. In detail, as shown in FIG. 4, the image extracting unit 15b compares the similarity of each in-vivo image in the in-vivo image group PG with the threshold value Th1 of the image-extraction condition and then extracts from the in-vivo image group PG in-vivo images P1-P6, P9, P11 to P13, P16, P17, and P19 to P21 whose similarities are lower than or equal to the threshold value Th1. The control unit 15 controls the storage unit 14 as the storage unit 14 stores these in-vivo images P1 to P6, P9, P11 to P13, P16, P17, and P19 to P21 in the extracted-image folder 14b as the in-vivo images to be displayed in a list.

The display controller 15a controls the display unit 13 as the display unit 13 displays a list of these in-vivo images P1 to P6, P9, P11 to P13, P16, P17, and P19 to P21 extracted by the image extracting unit 15b. Under the control of the display controller 15a, the display unit 13 displays the predetermined number of images at a time from a list of in-vivo images P1 to P6, P9, P11 to P13, P16, P17, and P19 to P21.

When instruction information for changing the image-extraction condition is input from the input unit 12 to the control unit 15, the control unit 15 determines, based on the instruction information for changing the image-extraction condition that is input from the input unit 12, that there is an instruction for changing the image-extraction condition. In this case, the extraction-condition setting unit 15c changes in a hierarchical manner the image-extraction condition for in-vivo images based on the instruction information for changing the image-extraction condition, i.e., changing the above threshold value Th1 into a threshold value Th2. The threshold value Th2 is a threshold value for the similarity of in-vivo images and is higher than the threshold value Th1 described above.

The image extracting unit 15b extracts in-vivo images matching the post-change image-extraction condition set by the extraction-condition setting unit 15c from the in-vivo image group PG of the subject 1 that is stored in the in-vivo image folder 14a. In detail, as shown in FIG. 4, the image extracting unit 15b compares the similarity of each in-vivo image in the in-vivo image group with the threshold value Th2 of the post-change image-extraction condition and re-extracts in-vivo images P1 to P21 whose similarities are lower than the threshold value Th2 from the in-vivo image group PG. The control unit 15 controls the storage unit 14 as the storage unit 14 stores these in-vivo images P1 to P21 in the extracted-image folder 14b as in-vivo images that are to be displayed in a list on the post-change extraction condition. As a result, the in-vivo images to be displayed in a list are updated by the re-extracted in-vivo images P1 to P21.

The display controller 15a controls the display unit 13 as the display unit 13 displays the predetermined number of images at a time from a list of in-vivo images P1 to P21, which are re-extracted on the threshold value Th2 from the in-vivo image group PG as shown in FIG. 4. At the same time, the display controller 15a controls the display unit 13 as the display unit 13 highlights the in-vivo images P7, P8, P10, P14, P15, and P18 that are not extracted on the pre-change image-extraction condition (threshold value Th1) but are newly extracted on the post-change image-extraction condition (threshold value Th2) in the re-extracted in-vivo images P1 to P21.

In detail, the display controller 15a determines a range W of the image-extraction condition (Th1 < W ≤ Th2) based on the threshold value Th1 of the pre-change image-extraction condition and the threshold value Th2 of the post-change image-extraction condition. The display controller 15a compares each similarity of the in-vivo images P1 to P21 with the range W of the image-extraction condition. Based on results of the comparison, the display controller 15a identifies the in-vivo images P7, P8, P14, P15, and P18, whose similarities are higher than the threshold value Th1 and equal to or lower than the threshold value Th2, as new in-vivo images. The new in-vivo images are in-vivo images that are not extracted on the threshold value Th1 of the pre-change image-extraction condition but are newly extracted on the threshold value Th2 of the post-change image-extraction condition. The display controller 15a controls the display unit 13 as the display unit 13 displays a list of in-vivo images P1 to P21 with the new in-vivo images P7, P8, P10, P14, P15, and P18 being highlighted.

Under the control of the display controller 15a, the display unit 13 displays the old in-vivo images meeting the pre-change threshold value Th1 and displays the new in-vivo images meeting the post-change threshold value Th2 being highlighted in a different display pattern from that of the old in-vivo images. Thus, the display unit 13 displays the in-vivo images P1 to P21. In detail, as shown in FIG. 5, the display unit 13 highlights the new in-vivo images P7, P8, P10, P14, P15, and P18 with thick frames or the like in the in-vivo images P1 to P21. At the same time, the display unit 13 displays the old in-vivo images P1 to P6, P9, P11-P13, P16, and P17, which meet the pre-change threshold value Th1 and have been displayed, in an ordinary display pattern. Viewing a display-list window 13a, users, such as doctors and nurses, can easily identify the highlighted in-vivo images P7, P8, P10, P14, P15, and P18 as the new in-vivo images in a list of the in-vivo images being displayed.

Under the control of the display controller 15a, the display unit 13 displays a display-list window 13a. As shown in FIG. 5, a time bar 13b and a slider 13c for displaying a time position of one of the in-vivo images being displayed in a list are displayed in the display-list window 13a. The time bar 13b is a graphic bar for displaying all time positions for the in-vivo image group PG of the subject 1. The slider 13c moves along the time bar 13b and points to a time position on the time bar 13b corresponding to the selected in-vivo image selected from the in-vivo images (e.g., in-vivo images P1 to P18 in FIG. 5) being displayed in a list.

Furthermore, in FIG. 5, the display controller 15a sets the number of in-vivo images that are to be displayed at a given time in the display-list window 13a at eighteen images. Not limited to eighteen, the number of in-vivo images to be displayed in the display-list window 13a can be any number desired. In FIG. 5, the in-vivo images P19 to P21 in the in-vivo images P1 to P21 to be displayed in a list are not displayed. The display unit 13 can display the remaining in-vivo images P19 to P21 by scrolling through the images, moving the slider 13c, or changing the setting for the number of images to be displayed.

The image display apparatus 4 in accordance with the first embodiment hierarchically increases the image-extraction condition, for example, by changing the threshold value Th1 to the threshold value Th2 as described above and re-displays a list of the in-vivo images. Not limited to this, the image-extraction condition may be hierarchically decreased and then a list of in-vivo images is re-displayed.

When the extraction-condition setting unit 15c changes the image-extraction condition from the threshold value Th2 to the threshold value Th1 in a hierarchical manner, the image extracting unit 15b extracts, based on the post-change threshold value Th1, in-vivo images P1 to P6, P9, P11 to P13, P16, P17, and P19 to P21 from the in-vivo image group PG. In this case, the display controller 15a excludes in-vivo images P7, P8, P10, P14, P15, P18, which are in the in-vivo images P1 to P21 extracted based on the pre-change threshold value Th2 and do not meet the post-change image-extraction condition, from in-vivo images to be displayed in a list. The display controller 15a controls the display unit 13 as the display unit 13 displays a list of the in-vivo images P1-P6, P9, P11 to P13, P16, P17, and P19 to P21, which have been extracted by the image extracting unit 15b after the change of the image-extraction condition. None of the in-vivo images P1 to P6, P9, Pull to P13, P16, P17, and P19 to P21 is an in-vivo image newly extracted on the post-change image-extraction condition. Therefore, the display controller 15a does not make the display unit 13 highlight in-vivo images.

As described above, in the first embodiment of the present invention, in-vivo images that are extracted on the image-extraction condition from the obtained in-vivo image group of the subject are displayed on the display unit. When the image-extraction condition is changed in a hierarchical manner, the in-vivo images to be displayed in a list are re-extracted on the post-change image-extraction condition. In these re-extracted in-vivo images, those in-vivo images which are newly extracted on the post-change image-extraction condition are highlighted. Thus, the old in-vivo images extracted on the pre-change image-extraction condition and the new in-vivo images newly extracted are displayed in different display patterns. As described, the in-vivo images are displayed in a list. In this manner, new in-vivo images can be easily visually distinguished from old in-vivo images in the in-vivo images being displayed on a screen. In the in-vivo images, the in-vivo images that have been observed are not to be mistakenly observed again. As a result, the image display apparatus can reduce the burden of observing each in-vivo image in a list displayed on a screen and allows users to efficiently observe the in-vivo images in a short time.

### Second Embodiment

A second embodiment of the present invention is described. In the first embodiment described above, in in-vivo images to be displayed in a list, those in-vivo images that are newly extracted on the post-change image-extraction condition are highlighted and displayed so that the new in-vivo images are displayed in a different display pattern from that of old in-vivo images. In the second embodiment, the new in-vivo images meeting the post-change extraction condition are displayed in a larger size than the old in-vivo images so that the new in-vivo images are displayed in a different display pattern from that of the old in-vivo images.

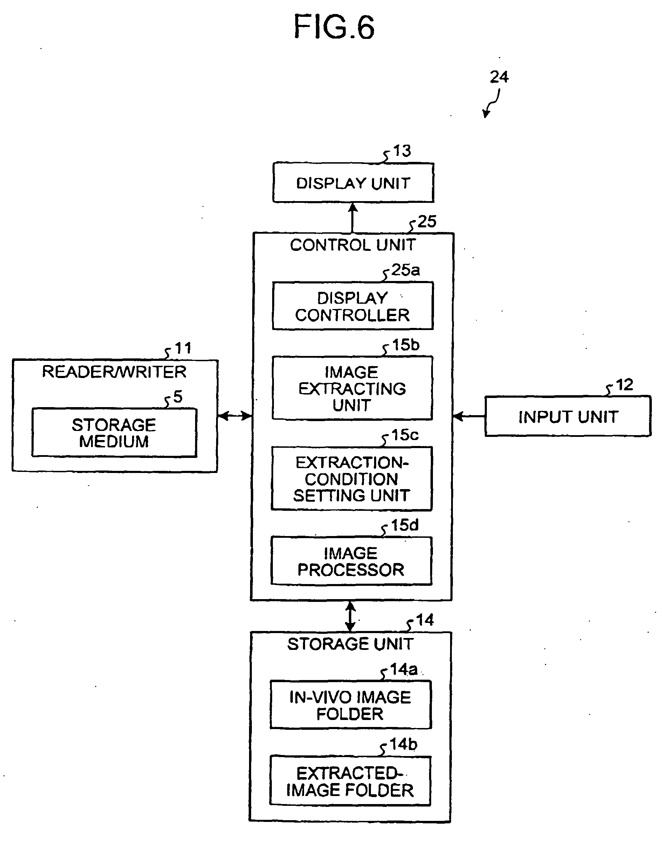

FIG. 6 is a schematic block diagram of an exemplary configuration of an image display apparatus in accordance with the second embodiment of the present invention. As shown in FIG. 6, an image display apparatus 24 in accordance with the second embodiment includes a control unit 25 instead of the control unit 15 of the image display apparatus 4 in accordance with the first embodiment described above. The control unit 25 includes a display controller 25a instead of the display controller 15a of the image display apparatus 4 in accordance with the first embodiment described above. An in-vivo information obtaining system in accordance with the second embodiment includes the image display apparatus 24 instead of the image display apparatus 4 of the in-vivo information obtaining system (see FIG. 1) in accordance with the first embodiment described above. Other configurations are the same as those of the first embodiment, and the same components have the same numerals.

As described, the control unit 25 includes the display controller 25a instead of the display controller 15a of the image display apparatus 4 in accordance with the first embodiment described above. Other than the functions of the display controller 25a, the control unit 25 has the same functions as those of the control unit 15 in accordance with the first embodiment described above.

When the image-extraction condition is changed in a hierarchical manner by the extraction-condition setting unit 15c, the display controller 25a makes the display unit 13 display a list of in-vivo images in a manner such that the display size of new in-vivo images that are newly extracted on the post-change image-extraction condition is larger than that of old in-vivo images that are extracted on the pre-change image-extraction condition. In this case, the display controller 25a controls the display unit 13 as the display unit 13 displays in-vivo images extracted by the image extracting unit 15b on the post-change extraction condition (i.e., in-vivo images to be displayed in a list) in a manner such that the display size of new in-vivo images remains normal and the display size of old in-vivo images is reduced. In the in-vivo images, the display size of the new in-vivo images is larger than that of the old in-vivo images and thus the display controller 25a displays the new in-vivo images in a different display pattern from that of the old in-vivo images. Other than the functions for making the display unit 13 display the new in-vivo images at a size larger than that of the old in-vivo images, the display controller 25a has the same functions as those of the display controller 15a of the image display apparatus 4 in accordance with the first embodiment described above.

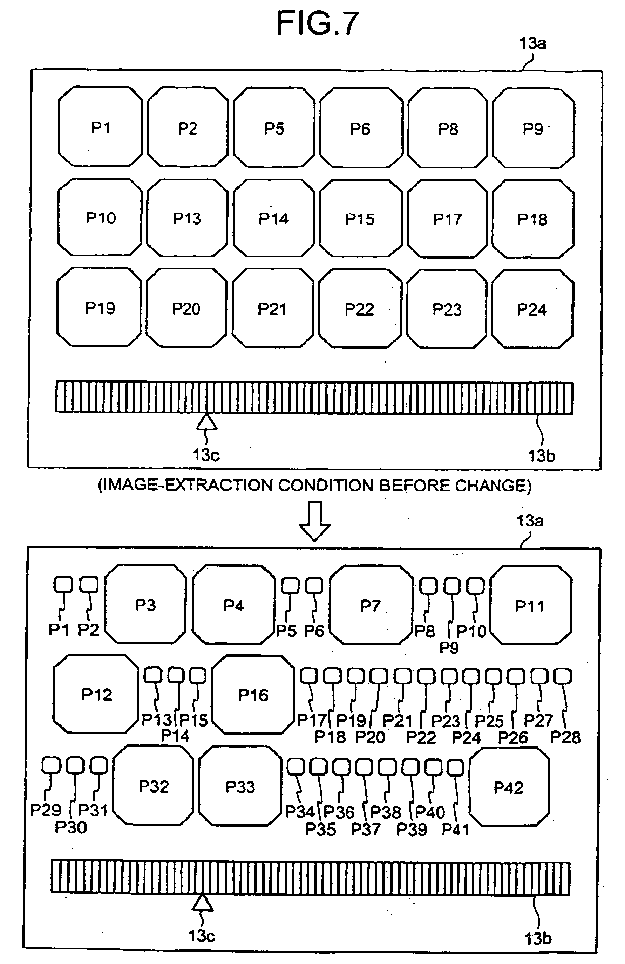

Operations of the image displays apparatus 24 in accordance with the second embodiment of the present invention are described. FIG. 7 is a schematic diagram illustrating a display pattern for a list of in-vivo images used by an image display apparatus 24 in accordance with the second embodiment of the present invention. FIG. 7 illustrates an example case where in-vivo images to be displayed in the display-list window 13a are changed because of the change of the image-extraction condition.

Other than the operations for displaying new in-vivo images in a different display pattern from that of old in-vivo images in in-vivo images extracted on the post-change extraction condition, the image display apparatus 24 in accordance with the second embodiment operates in the same manner as the image display apparatus 4 in accordance with the first embodiment described above. Therefore, the control unit 25 of the image display apparatus 24 has a different procedure from that of the first embodiment at Step S107 out of Steps S101 to S108 shown in FIG. 3. At the remaining Steps S101 to S106 and S108, the control unit 25 performs the same procedure as the first embodiment. Operations of the image display apparatus 24 at Step S107 are described below with reference to FIG. 7.

At Step S107 described above, the control unit 25 makes the display unit 13 display a list of in-vivo images in a manner such that the display size of new in-vivo images that are newly extracted on the post-change image extraction condition is larger than that of old in-vivo images extracted on the pre-change image-extraction condition. In this case, the display controller 25a controls the display unit 13 as the display unit 13 displays a predetermined number of in-vivo images at a given time from a list of in-vivo images extracted by the image extracting unit 15b on the post-change image-extraction condition. At the same time, the display controller 25a controls the display unit 13 as the display unit 13 displays the in-vivo images that are newly extracted on the post-change image-extraction condition at a normal size and the old in-vivo images extracted on the pre-change extraction condition at a reduced size. Similarly to the display controller 15a in accordance with the first embodiment, the display controller 25a identifies new in-vivo images or old in-vivo images from the in-vivo images based on the range of the image-extraction condition.

Similarly to the first embodiment, the display unit 13 of the image display apparatus 24 in accordance with the second embodiment displays a display-list window 13a. Under the control of the display controller 25a, the display unit 13 displays on the display-list window 13a a list of in-vivo images P1, P2, P5, P6, P8 to P10, P13 to P15, and P17 to P24 in the in-vivo images extracted on the pre-change image-extraction condition (see FIG. 7). The display unit 13 can display the remaining in-vivo images of the in-vivo images by scrolling through the images, moving the slider 13c, or changing the setting for the number of images to be displayed.

After the image-extraction condition is changed, the display unit 13, under the control of the display controller 25a at Step S107 described above, displays on the display-list window 13a a list of in-vivo images P1 to P42 of the in-vivo images extracted on the post-change image-extraction condition (see FIG. 7). In this case, in the in-vivo images P1 to P42, the display unit 13 displays the in-vivo images according to instructions for a display from the display controller 25a, i.e., the old in-vivo images P1, P2, P5, P6, P8 to P10, P13 to P15, P17 to P31, and P34 to P41 that are extracted on the pre-change image-extraction condition at a reduced size. At the same time, in the in-vivo images P1 to P42, the display unit 13 displays the new in-vivo images P3, P4, P7, P11, P12, P16, P32, P33, and P42 that are newly extracted on the post-change image-extraction condition at a normal size. The display unit 13 can display the remaining in-vivo images of the in-vivo images P1 to P42 by scrolling through images, moving the slider 13c, or changing the setting for the number of images to be displayed.

Viewing the display-list window 13a, users, such as a doctor and a nurse, can easily notice, in the in-vivo images P1 to P42 displayed in a list, the in-vivo images P3, P4, P7, P11, P12, P16, P32, P33, and P42 being displayed at a larger size than that of old in-vivo images as the new in-vivo images.

The image display apparatus 24 in accordance with the second embodiment may hierarchically increase the image-extraction condition and re-display a list of in-vivo images or hierarchically decrease the image-extraction condition and re-display a list of in-vivo images. In whichever cases, the display controller 25a controls the display unit 13 as the display unit 13 displays old in-vivo images meeting the pre-change extraction condition at a reduced size. Thus, in the in-vivo images to be displayed in a list, the display size of old in-vivo images are smaller than that of the new in-vivo images (i.e., smaller than a normal size).

Furthermore, the display controller 25a may control the display unit 13 as the display unit 13 deactivates the reduced display of in-vivo images based on the instruction information that is input from the input unit 12. In this case, under the control of the display controller 25a, the display unit 13 changes the display size of old in-vivo images back to the normal display size and displays the list of in-vivo images.

As described above, in the second embodiment of the present invention, a list of in-vivo images is described in a manner such that, in in-vivo images extracted from the in-vivo image group on the post-change image-extraction condition, new in-vivo images that are newly extracted on the post-change image-extraction condition are displayed in a larger display size than that of old in-vivo images that are extracted on the pre-change image-extraction condition. Other configurations are the same as those of the first embodiment. Therefore, the image display apparatus can provide the same operation benefits as those of the first embodiment and can highlight the new in-vivo images more remarkably in the in-vivo images to be displayed in a list. As a result, the image display apparatus allows users to tell unobserved in-vivo images from observed in-vivo images more easily.

### Third Embodiment

A third embodiment of the present invention is described. In the first embodiment described above, in-vivo images that do not meet the post-change image-extraction condition are removed from a display list. In the third embodiment, mark information indicating positions of those images that are removed from a display list is displayed in space among the in-vivo images being displayed in a list.

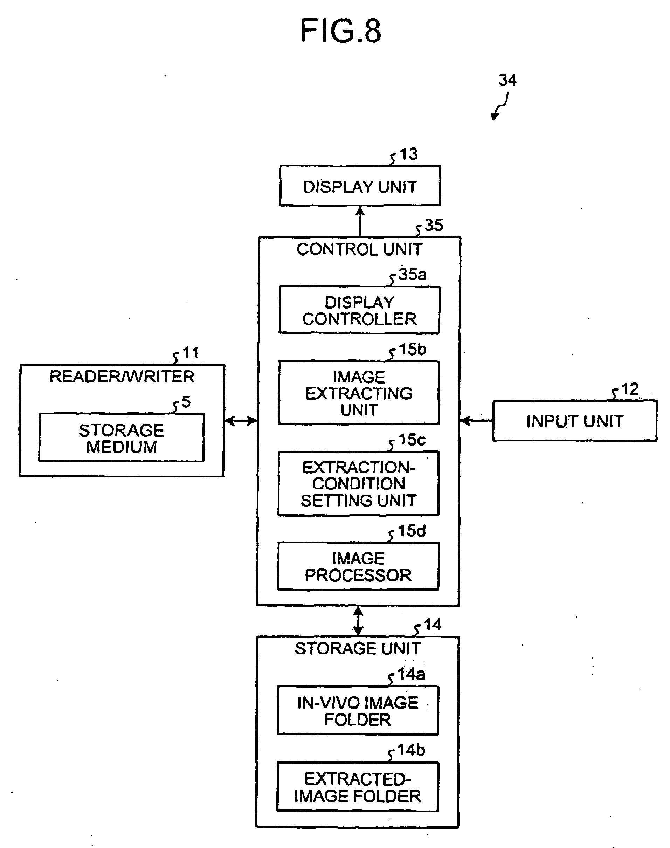

FIG. 8 is a schematic block diagram of an exemplary configuration of an image display apparatus in accordance with the third embodiment of the present invention. As shown in FIG. 8, an image display apparatus 34 in accordance with the third embodiment includes a control unit 35 instead of the control unit 15 of the image display apparatus 4 in accordance with the first embodiment described above. The control unit 35 includes a display controller 35a instead of the display controller 15a of the image display apparatus 4 in accordance with the first embodiment described above. An in-vivo information obtaining system in accordance with the third embodiment includes the image display apparatus 34 instead of the image display apparatus 4 the in-vivo information obtaining system (see FIG. 1) in accordance with the first embodiment described above. Other configurations are the same as those of the first embodiment, and the same components have the same numerals.

As described, the control unit 35 includes the display controller 35a instead of the display controller 15a of the image display apparatus 4 in accordance with the first embodiment described above. Other than the functions of the display controller 35a, the control unit 35 has the same functions as the control unit 15 in accordance with the first embodiment described above.

When the image-extraction condition is changed in a hierarchical manner by the extraction-condition setting unit 15c, the display controller 35a controls the display unit 13 as the display unit 13 displays mark information indicating the positions of the in-vivo images that do not meet the post-change image-extraction condition and are therefore removed from a display list. In detail, the image extracting unit 15b described above removes from a display list in-vivo images that are in-vivo images extracted on the pre-change image-extraction condition, i.e., in-vivo images in the previous display list, but do not meet the post-change image-extraction condition. Then, the display controller 35a controls the display unit 13 as the display unit 13 displays mark information, indicating the positions of those in-vivo images that are removed from the display list by the image extracting unit 15b based on post-change the image-extraction condition, in space among the in-vivo images in the current display list. Other than the functions of the display unit 13 for displaying the mark information indicating positions of the in-vivo images that are removed, the display controller 35a has the same functions as those of the display controller 15a of the image display apparatus 4 in accordance with the first embodiment described above.

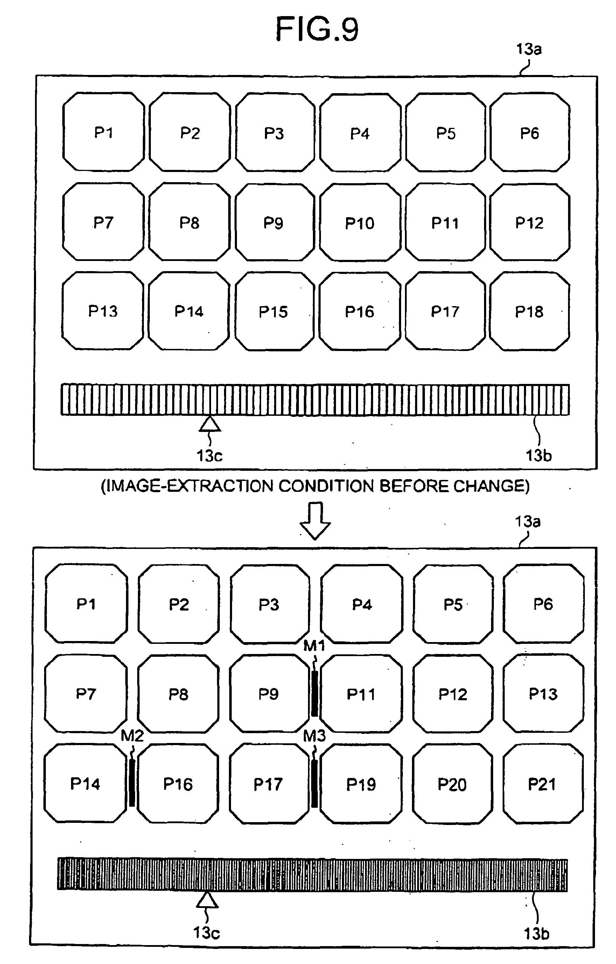

Operations of the image display apparatus 34 in accordance with the third embodiment of the present invention are described. FIG. 9 is a schematic diagram illustrating a display pattern for a list of in-vivo images used by an image display apparatus in accordance with the third embodiment of the present invention. FIG. 9 shows an example case where in-vivo images to be displayed in the display-list window 13a are changed after the change of the image-extraction condition.

Other than the operations for making the display unit 13 display marks indicating positions of the in-vivo images that are removed from a display list when the image-extraction condition is changed in a hierarchical manner, the image display apparatus 34 in accordance with the third embodiment is operated in the same manner as the image display apparatus 4 in accordance with the first embodiment described above. The control unit 35 of the image display apparatus 34 performs the procedure from Step S101 to S108 shown in FIG. 3 substantially in the same manner as the first embodiment. When there are in-vivo images removed from a display list at Step S106, the control unit 35 makes the display unit 13 display mark information indicating the positions of the removed in-vivo images at Step S107 described above. Referring to FIG. 9, the following describes operations of the image display apparatus 34 at Step S107 in a case where there is at least one of the in-vivo images that meets the pre-change image-extraction condition but is removed from the display list at Step S106.

At Step S107 described above, the control unit 35 makes the display unit 13 display marks indicating the positions of the in-vivo images that are removed from the display list based on the post-change image-extraction condition. In this case, similarly to the first embodiment described above, the display controller 35a controls the display unit 13 as the display unit 13 display a list of in-vivo images with new in-vivo images being highlighted. Based on each of frame numbers of in-vivo images in the current display list and frame numbers of the removed in-vivo images, the display controller 35a identifies spaces among images corresponding to the removed in-vivo images. The display controller 35a controls the display unit 13 as the display unit 13 displays mark information, indicating the positions of the removed in-vivo images, in the identified spaces among images.

When no in-vivo image is removed from the display list after the change of the image-extraction condition, i.e., when all in-vivo images meeting the pre-change image-extraction condition are extracted at Step S106 based on the post-change image-extraction condition, the control unit 35 performs the same procedure at Step S107 as the first embodiment described above.

Before the change of the image-extraction condition, the display unit 13 of the image display apparatus 34 in accordance with the third embodiment under the control of the display controller 35a displays in the display-list window 13a a list of in-vivo images P1 to P18 in is-vivo images extracted on the pre-change extraction condition (see FIG. 9). The display unit 13 can display the remaining in-vivo images other than the in-vivo images by scrolling through images, moving the slider 13c, or changing the setting for the number of images to be displayed.

After the change of the image-extraction condition, the display unit 13 under the control of the display controller 15a at Step S107 displays in the display list-window 13a a list of in-vivo images P1 to P9, P11 to P14, P16, P17, and P19 to P21 (see FIG. 9) that are extracted on the post-change image-extraction condition. Furthermore, the display unit 13 displays in the display-list window 13a marks M1, M2, and M3 that respectively correspond to in-vivo images P10, P15, and P18 that are removed from the display list by the image extracting unit 15b described above based on the post-change image-extraction condition.

In detail, the mark M1 indicates the position of the in-vivo image P10 that is removed from the display list and is displayed in the space where the removed in-vivo image P10 is located, i.e., the space between the in-vivo image P9 and the in-vivo image P11. The mark M2 indicates the position of the in-vivo image P15 that is removed from the display list and is displayed in the space where the removed in-vivo image P15 is located, i.e., the space between the in-vivo image P14 and the in-vivo image P16. The mark M3 indicates the position of the in-vivo image P18 that is removed from the display list and is displayed in the space where the removed in-vivo image P18 is located, i.e., the space between the in-vivo image P17 and the in-vivo image P19.

The display unit 13 can display the in-vivo images other than the in-vivo images P1 to P9, P11 to P14, P16, P17, and P19 to P21 by scrolling through images, moving the slider 13c, or changing the setting for the number of images to be displayed.

Viewing the display-list window 13a, users, such as doctors and nurses, can identify new in-vivo images more easily from a list of in-vivo images being displayed and, furthermore, can visually identify positions of removed in-vivo images with information indicating that some in-vivo images are removed from the display list after the change of the image-extraction condition.

The image display apparatus 34 in accordance with the third embodiment may hierarchically increase the image-extraction condition and re-display a list of in-vivo images or hierarchically decrease the image-extraction condition and re-display a list of in-vivo images. In whichever cases, the display controller 35a controls the display unit 13 as the display unit 13 displays mark information indicating the position of the in-vivo images that are removed from the display list based on the post-change image-extraction condition.

When at least one of the in-vivo images meeting the pre-change image-extraction condition is removed from the display list based on the post-change image-extraction condition in the third embodiment of the present invention described above, a list of in-vivo images is displayed along with mark information indicating the positions of the removed in-vivo images. Other configurations are the same as those of the first embodiment. Therefore, the image display apparatus can provide the same operation benefits as the first embodiment and can allow users to visually identify the positions of the removed in-vivo images with information indicating that some in-vivo images are removed from the display list after the change of the image-extraction condition. As a result, the image display apparatus allows users to check a displayed list of in-vivo images more easily in relation to the image-extraction condition.

### Fourth Embodiment

A fourth embodiment of the present invention is described. In the first embodiment, new in-vivo images that are newly extracted on the post-change image-extraction condition are highlighted. In the fourth embodiment, the image-extraction condition for in-vivo images to be displayed is determined for each image, and level information indicating a determined level of the image-extraction condition is displayed for each image.

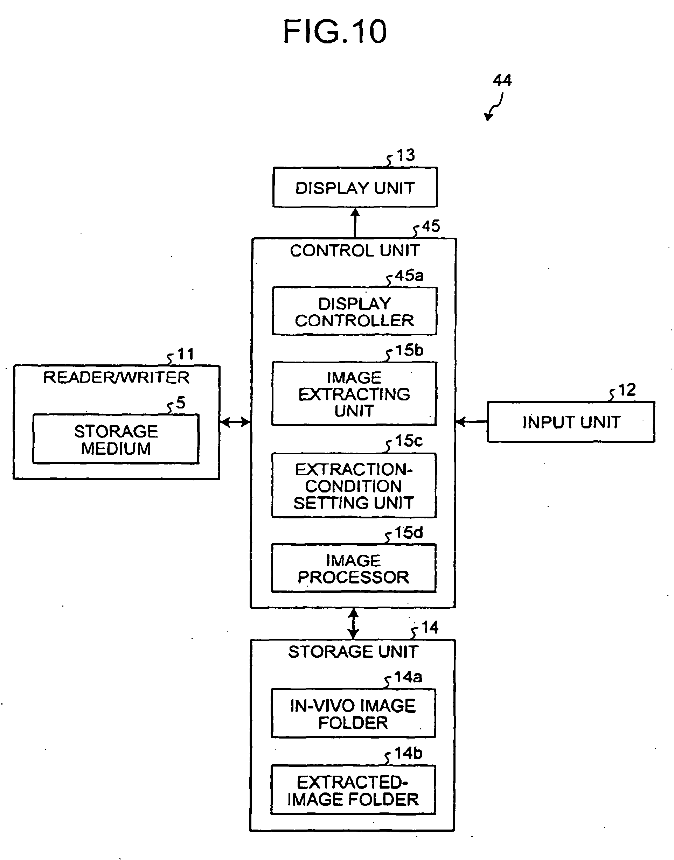

FIG. 10 is a schematic block diagram of an exemplary configuration of an image display apparatus in accordance with the fourth embodiment of the present invention. As shown in FIG. 10, an image display apparatus 44 in accordance with the fourth embodiment includes a control unit 45 instead of the control unit 15 of the image display apparatus 4 in accordance with the first embodiment described above. The control unit 45 includes a display controller 45a instead of the display controller 15a of the image display apparatus 4 in accordance with the first embodiment described above. An in-vivo information obtaining system in accordance with the fourth embodiment includes the image display apparatus 44 instead of the image display apparatus 4 of the in-vivo information obtaining system (see FIG. 1) in accordance with the first embodiment described above. Other configurations are the same as those of the first embodiment, and the same components have the same numerals.

As described above, the control unit 45 includes the display controller 45a instead of the display controller 15a of the image display apparatus 4 in accordance of the first embodiment described above. Other than the functions of the display controller 45a, the control unit 45 has the same functions as the control unit 15 in accordance with the first embodiment described above.

When the image-extraction condition is changed in a hierarchical manner by the extraction-condition setting unit 15c, the display controller 45a makes the display unit 13 determine a level of the image-extraction condition for each in-vivo image extracted after the change of the image-extraction condition and display each extracted in-vivo image along with the determined level information of the image-extraction condition. In this case, the display controller 45a compares the similarity of each in-vivo image to be displayed in a list with the predetermined range of the image-extraction condition. Based on'a result of the comparison, the display controller 45a determines the level of the image-extraction condition for each in-vivo image. The display controller 45a controls the display unit 13 as the display unit 13 displays a list of in-vivo images to be displayed in a manner such that the level information indicating the determined level of the image-extraction condition is displayed for each in-vivo image being displayed in a list. The display controller 45a makes the display unit 13 display level information of the image-extraction condition for each in-vivo image being displayed in a list so that new in-vivo images are displayed in a different display pattern from that of old in-vivo images in the in-vivo images being displayed in a list. Other than the functions for determining the level information of the image-extraction condition for each in-vivo image and making the display unit 13 display the level information, the display controller 45a has the same functions as the display controller 15a of the image display apparatus 4 in accordance with the first embodiment described above.

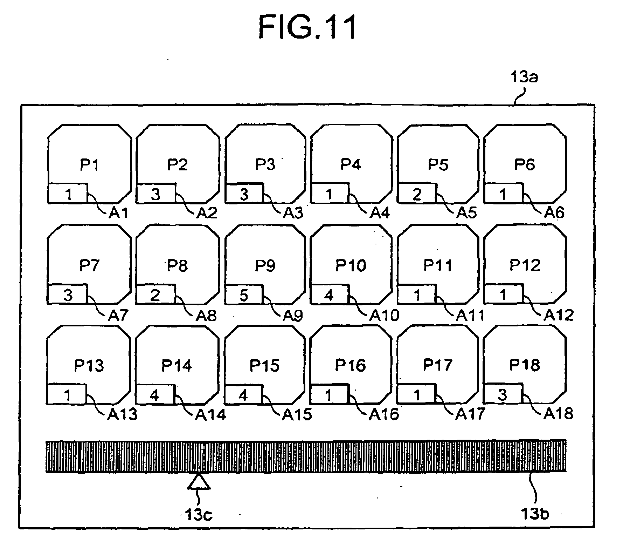

Operations of the image display apparatus 44 in accordance with the fourth embodiment of the present invention are described. FIG. 11 is a schematic diagram illustrating a display pattern for displaying a list of in-vivo images with level information of the image-extraction condition used by an image display apparatus in accordance with the fourth embodiment of the present invention. FIG. 11 illustrates the display-list window 13a in which the level information of the image-extraction condition is displayed for each in-vivo image in the in-vivo images to be displayed in a list.

Other than the operations for displaying the level information of the image-extraction condition for each in-vivo image to be displayed in a list, the image display apparatus 44 in accordance with the fourth embodiment is operated in the same manner as the image display apparatus 4 in accordance with the first embodiment described above. In the Steps S101 to S108 shown in FIG. 3, the control unit 45 in the image display apparatus 44 performs a different procedure at Step S107 from that of the first embodiment. At the remaining Steps S101 to S106 and S108, the control unit 45 performs the same procedure as the first embodiment. The operations of the image display apparatus 44 at Step S107 are described below with reference to FIG. 11.

At Step S107 described above, the control unit 45 appends level information of the image-extraction condition to each in-vivo image extracted on the post-change image-extraction condition and makes the display unit 13 display the in-vivo images. In this case, the display controller 45a controls the display unit 13 as the display unit 13 displays a predetermined number of in-vivo images at a time from the in-vivo images that are re-extracted on the post-change image-extraction condition by the image extracting unit 15b as described above. Furthermore, the display controller 45a determines the level of the image-extraction condition for each of the in-vivo images. The display controller 45a controls the display unit 13 as the display unit 13 displays the in-vivo images with the appended level information of the image-extraction condition that is determined for each in-vivo image.

In detail, the display controller 45a sets ranges of the image-extraction condition at multi levels in association with the grades of the image-extraction condition that are set by the extraction-condition setting unit 15c described above. For example, based on the threshold values Th11 to Th15 of the image-extraction condition that is set in a hierarchical manner by the extraction-condition setting unit 15c described above, the display controller 45a sets five-grade ranges w1 to W5 of the image-extraction condition. The threshold values Th11 to Th15 are calculated by the image processor 15d described above based on the similarities of in-vivo images. The magnitude relation for the threshold values Th11 to Th15 is Th15 < Th14 < Th13 < Th12 < Th11. The display controller 45a sets the five-grade ranges W1 to W5 of the image-extraction condition based on the threshold values Th11 to TH15 of the image-extraction condition.

In detail, the display controller 45a sets the range W1 of the image-extraction condition as the level 1 range of the image-extraction condition, which is the lowest image-extraction level for in-vivo images; W1 includes the image-extraction condition that is higher than the threshold value Th12 and lower than or equal to the threshold value Th11. The display controller 45a sets the range W2 of the image-extraction condition as the level 2 range of the image-extraction condition, which is the next higher level to the level 1; W2 includes the image-extraction condition that is higher than the threshold value Th13 and lower than or equal to the threshold value Th12. The display controller 45a sets the range W3 of the image-extraction condition as the level 3 range of the image-extraction condition, which is the next higher level to the level 3; W3 includes the image-extraction condition that is higher than the threshold value Th14 and lower than or equal to the threshold value Th13. The display controller 45a sets the range W4 of the image-extraction condition as the level 4 range of the image-extraction condition, which is the next higher level to the level 4; W4 includes the image-extraction condition that is higher than the threshold value Th15 and lower than or equal to the threshold value Th14. The display controller 45a sets the range W5 of the image-extraction condition as the level 5 range of the image-extraction condition, which is the next higher level to the level 5; W5 includes the image,-extraction condition that is lower than or equal to the threshold value Th15.

For the in-vivo images re-extracted on the post-change extraction condition by the image extracting unit 15b, the display controller 45a determines the level of the image-extraction condition for each in-vivo image in the in-vivo images by comparing the similarity of each in-vivo image with the ranges W1 to W5 of the image-extraction condition. When the similarity of the in-vivo image is within the range W1 of the image-extraction condition, the display controller 45a determines that the level of the image-extraction condition of the in-vivo image is level 1. When the similarity of the in-vivo image is within the range W2 of the image-extraction condition, the level of the image-extraction condition of the in-vivo image is level 2. When the similarity of the in-vivo image is within the range W3 of the image-extraction condition, the level of the image-extraction condition of the in-vivo image is level 3. When the similarity of the in-vivo image is within the range W4 of the image-extraction condition, the level of the image-extraction condition of the in-vivo image is level 4. When the similarity of the in-vivo image is within the range W5 of the image-extraction condition, the level of the image-extraction condition of the in-vivo image is level 5.

The display controller 45a controls the display unit 13 as the display unit 13 displays each in-vivo image to be displayed in a list with the appended level information indicating the level of the image-extraction condition that is determined as described above. The level information is, for example, a numeric value indicating the level of the image-extraction condition. Under the control of the display controller 45a, the level information is overlapped with each in-vivo image being displayed in a list.

Under the control of the display controller 45a described above, the display unit 13 of the image display apparatus 44 in accordance with the fourth embodiment displays in the display-list window 13a a list of in-vivo images P1 to P18 that are extracted on the post-change image-extraction condition (see FIG. 11). The display unlit 13 can display the remaining in-vivo images other than the in-vivo images P1 to P18 by scrolling through images, moving the slider 13c, or changing the setting for the number of images to be displayed.

Under the control of the display controller 25a described above, the display unit 13 displays in the display-list window 13a level information A1 to A18 of the image-extraction condition along with the in-vivo images P1 to P18. In this case, as shown in FIG. 11, the display unit 13 displays a list of in-vivo images with the level information A1 to A18 overlapped with the sides and areas nearby of those in-vivo images P1 to P18 being displayed. The level information A1 to A18 indicates the level of the image-extraction condition for the in-vivo images P1 to P18 as the numeric value. For example, the level information A1 appended to the in-vivo image P1 indicates that the level of the image-extraction condition of the in-vivo image P1 is "level 1", and the level information A9 appended to the in-vivo image P9 indicates that the level of the image-extraction condition of the in-vivo image P9 is "level 5".

The display unit 13 displays the in-vivo images P1 to P18 with the appended level information A1 to A18. Thus, the display unit 13 displays the in-vivo images in different display patterns depending on the level of the image-extraction condition. For example, when the image-extraction condition is changed into the threshold value Th11, the display unit 13 appends the level information A1, A4, A6, A11, A12, A13, A16, and A17 indicating "level 1" to the new in-vivo images P1, P4, P6, P11, P12, P13, P16, and P17 extracted on the post-change image-extraction condition, respectively, and displays the in-vivo images. As a result, the new in-vivo images P1, P4, P6, P11, P13, P16, and P17 are displayed in a different display pattern from the old in-vivo images P2, P3, P5, P7, P8, P10, P14, P15, and P18 to which level information indicating different levels from the level 1 is appended.

Viewing the display-list window 13a, users, such as a doctor and a nurse, can identify new in-vivo images and old in-vivo images more easily from the in-vivo images P1 to P18 being displayed in a list and can easily check the levels of the image-extraction conditions of the in-vivo images P1 to P18. As a result, users can check the level of the image-extraction condition that is suitable for observing the in-vivo images. Based on the check, the users can easily set the image-extraction condition in the image display apparatus 44 at the level suitable for observing the in-vivo images.

The image display apparatus 44 in accordance with the fourth embodiment may hierarchically increase the image-extraction condition and re-display a list of in-vivo images or may hierarchically decrease the image-extraction condition and re-display a list of in-vivo images. In whichever cases, the display controller 45a determines the level of the image-extraction condition for each in-vivo image to be displayed in a list and makes the display unit 13 display a list of the in-vivo images with the level information of the image-extraction condition being appended to each in-vivo image being displayed in a list.

Furthermore, the display controller 45a may control the display unit 13 at Step S103 as the display unit 13 display the level information of the image-extraction condition with each in-vivo image being displayed in a list. In this case, the display controller 45a may determine the level of the image-extraction condition for each in-vivo image to be displayed in a list and control the display unit 13 as the display unit 13 displays a list of in-vivo images with the level information of the image-extraction condition being appended to each in-vivo image being displayed in a list.

As described above, in the fourth embodiment of the present invention, the level of the image-extraction condition is determined for each in-vivo image that is extracted to be displayed in a list. A list of the in-vivo images is displayed with the level information of the image-extraction condition being appended to each in-vivo image. Other configurations are the same as the first embodiment. Therefore, the image display apparatus can provide the same operation benefits and allow users to easily view the level of the image-extraction condition of each in-vivo image being displayed in a list. As a result, the image display apparatus allows users to easily set the level of the image-extraction condition that is suitable for observing the in-vivo images.

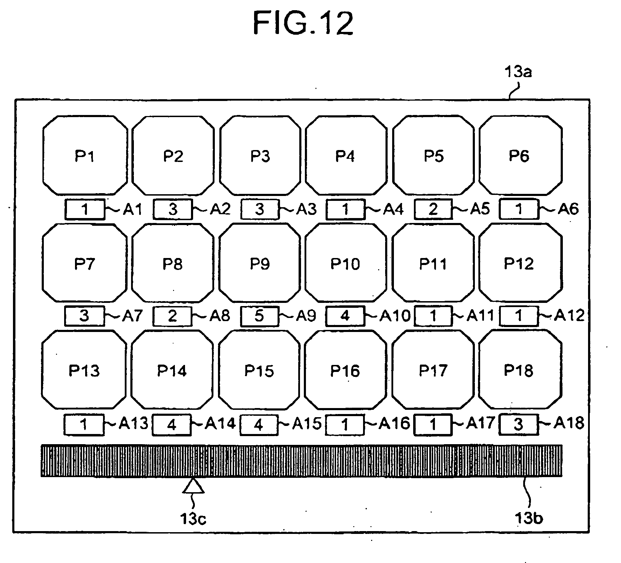

In the fourth embodiment described above, the level information of the image-extraction condition is overlapped with each in-vivo image being displayed in a list. Not limited to this, the level information of the image-extraction condition may not be overlapped with each in-vivo image as long as the level information is displayed in association with each in-vivo image being displayed in a list. FIG. 12 is a schematic diagram illustrating a variation of level information of an image-extraction condition that is displayed together with in-vivo images being displayed in a list. As shown in the level information A1 to A18 in FIG. 12, the level information of the image-extraction condition may be appended to a nearby part outside the in-vivo images being displayed in a list.

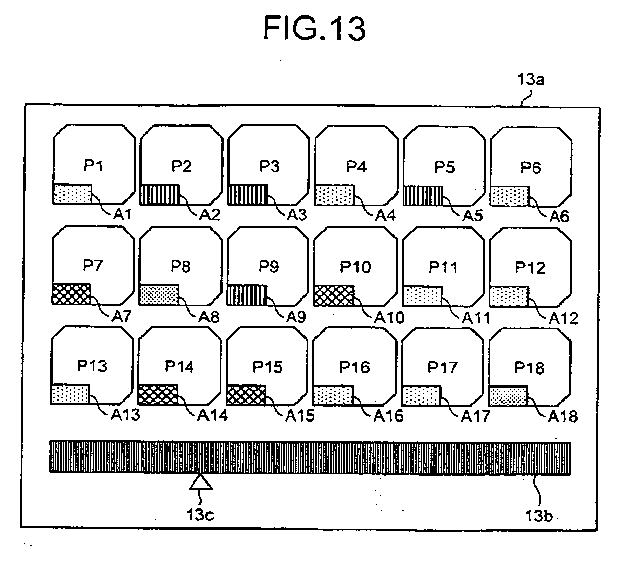

Furthermore, in the fourth embodiment described above, the level information indicating the level of the image-extraction condition as a numeric value is appended to the in-vivo images. Not limited to this, the level information of the image-extraction condition indicating the level of the image-extraction condition may be information other than a numeric value. FIG. 13 is a schematic diagram illustrating another variation of level information of an image-extraction condition that is displayed together with in-vivo images to be displayed in a list. As shown in the level information A1 to A18 in FIG. 13, the level information of the image-extraction condition may be mark information such as pattern and color that differs for each level of the image-extraction condition. The level of the image-extraction condition can be indicated by a display pattern such as pattern and color of the mark information.

Furthermore, in the first, second, third, and fourth embodiments described above, the threshold value for the similarity of the in-vivo image is set as the image-extraction condition for extracting in-vivo images to be displayed in a list and this value can be changed. Not limited to this, the image-extraction condition may not be the threshold value for the similarity of the in-vivo image as long as the image-extraction condition can be changed in a hierarchical manner. In detail, the image-extraction condition may be a threshold value for a predetermined-color level such as red and green in an in-vivo image, or may be a threshold value for the average-color level of an in-vivo image, or may be a predetermined-color spreading level (i.e., a number of predetermined-color pixels), or may be a frame interval for removing or extracting an in-vivo image from an in-vivo image group that is captured in time series, or may be a combination of two or more of these described above.

Furthermore, when the image-extraction condition of the in-vivo image is a combination of two or more of the color-level threshold value, the average-color level threshold value, the color-spreading level threshold value, or the frame intervals, the extraction-condition setting unit 15c described above changes the image-extraction condition set by the combination based on the input information from the input unit 12 in a hierarchical manner. For example, the extraction-condition setting unit 15c may increase or decrease the threshold value for a red-color level in an in-vivo image while not changing the threshold value for the similarity of the in-vivo image. For another example, the extraction-condition setting unit 15c may increase or decrease the threshold value for the similarity of an in-vivo image while not changing the frame intervals for removing the in-vivo images. In this case, the image extracting unit 15b, for example, extracts in-vivo images whose similarities are lower than or equal to the predetermined threshold value and then extracts, from the extracted in-vivo images, in-vivo images whose red-color levels are lower than or equal to the threshold value that has been increased or decreased. Furthermore, the image extracting unit 15b, for example, removes in-vivo images at predetermined intervals from the in-vivo image group and then extracts, from the in-vivo images being removed from the in-vivo image group, in-vivo images whose similarities are lower than or equal to the threshold value that has been increased or decreased.

In the first embodiment described above, outer frames of new in-vivo images in a list of the in-vivo images being displayed are made thick so that the new in-vivo images are highlighted. Not limited to this, one of the upper, bottom, right, and left sides may be made thick and highlighted or one of the upper, bottom, right, and left sides may be colored with a predetermined color and highlighted, or such a highlighting process may include a combination of these. Furthermore, a predetermined mark may be appended to the new in-vivo images so that the new in-vivo images are highlighted. Furthermore, the new in-vivo images may blink so as to be highlighted. In still another example, she remaining in-vivo images (i.e., old in-vivo images) other than the new in-vivo images may be colored with gray or other colors so that the new in-vivo images are relatively highlighted.

Furthermore, in the second embodiment described above, old in-vivo images in a list of the in-vivo images being displayed are displayed at a reduced size so that the display size of new in-vivo images is larger than that of the old in-vivo images. Not limited to this, new in-vivo images in the in-vivo images may be displayed at an enlarged size. Furthermore, new in-vivo images may be displayed at an enlarged size and old in-vivo images may be displayed at a reduced size. Furthermore, the display controller described above may set an enlargement rate of new in-vivo images or a reduction rate of old in-vivo images based on information input from the input unit 12.

Furthermore, in the third embodiment, mark information indicating positions of in-vivo images removed from a display list is displayed along with a list of in-vivo images being displayed. A numeric values indicating the numbers of removed in-vivo images may be appended to the mark information, and the mark information indicating the positions and the number of the removed in-vivo images may be displayed in spaces among the in-vivo images. As a result, users can easily view the positions and the number of the removed in-vivo images.

Furthermore, in the third embodiment described above, new in-vivo images are highlighted similarly to the first embodiment. Not limited to this, the image display apparatus in accordance with the third embodiment may display in-vivo images in a manner such that the display size of new in-vivo images is larger than that of old in-vivo images similarly to the second embodiment or that the level information of the image-extraction condition is appended to each in-vivo image similarly to the fourth embodiment. The image display apparatus of the present invention may be a combination of the second and third embodiments described above or a combination of the third and fourth embodiments described above.

In the first, second, third and fourth embodiments described above, operations of the image display apparatus, i.e., image display methods, are performed by a computer. Image display programs can be read and executed by the image display apparatus. The image display programs may be stored in a storage medium, e.g., a CD-ROM.

The invention is defined by the appended claims.

## Claims

1. An image display apparatus (4; 24; 34; 44), comprising:
an image obtaining unit (11) that obtains a series of images captured in time series;
an extraction-condition setting unit (15c) that sets an image-extraction condition;
an image extracting unit (15b) that extracts images from a series of images based on the image-extraction condition set by the extraction-condition setting unit (15c); and
a display unit (13) that displays a list of images extracted by the image extracting unit (15b); **characterized in that**
the extraction-condition setting unit (15c) is adapted to change the image-extraction condition according to different threshold values, and
the image display apparatus (4; 24; 34; 44) further comprises
a display controller (15a; 25a; 35a; 45a) that controls the display unit (13) so that when the image-extraction condition is changed to different threshold values by the extraction-condition according setting unit (15c), new images that are newly extracted according to the post-change image-extraction condition, out of images extracted according to the image-extraction condition that is changed by the image extracting unit (15b) are displayed in a different display pattern from that of old images that have been extracted according to the pre-change image-extraction condition.

2. The image display apparatus (4; 24; 34; 44) according to claim 1, wherein
the display controller (15a; 25a; 35a; 45a) controls the display unit (13) so that the new images that are extracted according to the post-change image-extraction condition are highlighted and displayed.

3. The image display apparatus (4; 24; 44) according to claim 2, wherein
the display controller (15a; 25a; 45a) controls the display unit (13) so that the new images at a larger size than that of the old images are highlighted and displayed.

4. The image display apparatus (4; 34; 44) according to claim 1, wherein
the display controller (15a; 35a; 45a) determines a level of the image-extraction condition of each image included in the images and controls the display unit (13) so that the level information indicating the determined level is appended to each image and thus the new images are displayed in a different display pattern from that of the old images.

5. The image display apparatus (4; 24; 34; 44) according to any one of claims 1 to 4, wherein
the display controller (15a; 25a; 35a; 45a) controls the display unit (13) so that mark information indicating the positions of images that are removed from a display list according to the post-change image-extraction condition is displayed.

6. The image display apparatus (4; 24; 34; 44) according to claim 5, wherein
the display unit (13) displays the mark information in space among images being displayed in a list.

7. An image display method, comprising:
obtaining a series of images captured in time series;
setting an image-extraction condition;
extracting images from a series of images based on the image-extraction condition; and
displaying a list of extracted images; **characterized in that**
changing the image-extraction condition according to different threshold values, and
when the image-extraction condition is changed to different threshold values, new images that are newly extracted according to the post-change image-extraction condition, out of images extracted according to the image-extraction condition, are displayed in a different display pattern form that of old images that have been extracted according to the pre-change image-extraction condition.

8. The image display method according to claim 7, wherein
the displaying includes highlighting and displaying the new images that are newly extracted according to the post-change image-extraction condition, on the display unit (13).

9. The image display method according to claim 8, wherein
the displaying includes displaying on the display unit (13) the new images at a larger size than that of the old images so that the new images are highlighted.

10. The image display method according to claim 7, wherein
the displaying includes determining a level of the image-extraction condition of each image included in the images is determined, and appending level information indicating the determined level to each image so that the new images are displayed in a different display pattern from that of the old images.

11. The image display program according to any one of claims 7 to 10, wherein
the displaying includes displaying on the display unit (13) mark information indicating the positions of images removed from a display list according to the post-change image-extraction condition.

12. The image display method according to claim 11, wherein
the displaying includes displaying the mark information in space among the images on the display unit (13).

13. An image display program that executes, when run on a computer, the steps of method claim 7.

## Patentansprüche

1. Bildanzeigevorrichtung (4; 24; 34; 44), mit:
einer Bilderlangungseinheit (11), die eine in Zeitserien aufgenommene Bilderserie erlangt;
einer Extraktionsbedingungs-Einstelleinheit (15c), die eine Bildextraktionsbedingung einstellt;
einer Bildextraktionseinheit (15b), die Bilder aus einer Bilderserie auf der Basis der von der Extraktionsbedingungs-Einstelleinheit (15c) eingestellten Bildextraktionsbedingung extrahiert; und
einer Anzeigeeinheit (13), die eine von der Bildextraktionseinheit (15b) extrahierte Liste von Bildern anzeigt; **dadurch gekennzeichnet, dass**
die Extraktionsbedingungs-Einstelleinheit (15c) die Bildextraktionsbedingung gemäß verschiedener Schwellenwerte zu ändern vermag, und
die Bildanzeigevorrichtung (4; 24; 34; 44) ferner umfasst eine Anzeige-Steuereinrichtung (15a; 25a; 35a; 45a), die die Anzeigeeinheit (13) so steuert dass, wenn die Bildextraktionsbedingung durch die Extraktionsbedingungs-Einstelleinheit (15c) zu verschiedenen Schwellenwerten verändert wird, neue Bilder, die entsprechend der Bildextraktionsbedingung nach der Änderung neu extrahiert werden aus Bildern, die entsprechend der von der Bildextraktionseinheit (15b) geänderten Bildextraktionsbedingung extrahiert werden, in einem anderen Anzeigemuster als dem von alten Bildern, die entsprechend der Bildextraktionsbedingung vor der Änderung extrahiert worden sind, angezeigt werden.

2. Bildanzeigevorrichtung (4; 24; 34; 44) nach Anspruch 1, wobei
die Anzeige-Steuereinrichtung (15a; 25a; 35a; 45a) die Anzeigeeinheit (13) so steuert, dass die neuen Bilder, die entsprechend der Bildextraktionsbedingung nach der Änderung extrahiert werden, hervorgehoben und angezeigt werden.

3. Bildanzeigevorrichtung (4; 24; 44) nach Anspruch 2, wobei
die Anzeige-Steuereinrichtung (15a; 25a; 45a) die Anzeigeeinheit (13) so steuert, dass die neuen Bilder mit einer größeren Größe als die der alten Bilder hervorgehoben und angezeigt werden.

4. Bildanzeigevorrichtung (4; 34; 44) nach Anspruch 1, wobei
die Anzeige-Steuereinrichtung (15a; 35a; 45a) eine Stufe der Bildextraktionsbedingung jedes in den Bildern enthaltenen Bildes bestimmt und die Anzeigeeinheit (13) so steuert, dass die die bestimmte Stufe angebende Stufen-Information an jedes Bild angehängt wird und so die neuen Bilder in einem zu den alten Bildern unterschiedlichen Anzeigemuster angezeigt werden.

5. Bildanzeigevorrichtung (4; 24; 34; 44) nach einem der Ansprüche 1 bis 4, wobei
die Anzeige-Steuereinrichtung (15a; 25a; 35a; 45a) die Anzeigeeinheit (13) so steuert, dass Markierungsinformation, welche die Positionen der Bilder angibt, die von einer Anzeigeliste gemäß der Bildextraktionsbedingung nach der Änderung entfernt werden, angezeigt wird.

6. Bildanzeigevorrichtung (4; 24; 34; 44) nach Anspruch 5, wobei
die Anzeigeeinheit (13) die Markierungsinformation in einem Zwischenraum zwischen Bildern anzeigt, die in einer Liste angezeigt werden.

7. Bildanzeigeverfahren, umfassend:
Erlangen einer in Zeitserien aufgenommenen Bilderserie;
Einstellen einer Bildextraktionsbedingung;
Extrahieren von Bildern aus einer Bilderserie auf der Basis der
Bildextraktionsbedingung; und
Anzeigen einer Liste extrahierter Bilder; **dadurch gekennzeichnet, dass** die Bildextraktionsbedingung gemäß verschiedenen Schwellenwerten verändert wird und, wenn die Bildextraktionsbedingung zu verschiedenen Schwellenwerten verändert wird, neue Bilder, die entsprechend der Bildextraktionsbedingung nach der Änderung aus gemäß der Bildextraktionsbedingung extrahierten Bildern neu extrahiert werden, in einem anderen Anzeigemuster angezeigt werden als das von alten Bildern, die gemäß der Bildextraktionsbedingung vor der Änderung extrahiert worden sind.

8. Bildanzeigeverfahren nach Anspruch 7, wobei
das Anzeigen ein Hervorheben und Anzeigen der neuen Bilder, die entsprechend der Bildextraktionsbedingung nach der Änderung neu extrahiert werden, an der Anzeigeeinheit (13) umfasst.

9. Bildanzeigeverfahren nach Anspruch 8, wobei
das Anzeigen ein Anzeigen der neuen Bilder an der Anzeigeeinheit (13) mit einer größeren Größe als der der alten Bilder umfasst, so dass die neuen Bilder hervorgehoben werden.

10. Bildanzeigeverfahren nach Anspruch 7, wobei
das Anzeigen ein Bestimmen der Stufe der Bildextraktionsbedingung jedes in den Bildern enthaltenen Bildes sowie das Anhängen von die bestimmte Stufe angebender Stufen-Information an jedes Bild umfasst, so dass die neuen Bilder in einem zu den alten Bildern unterschiedlichen Anzeigemuster angezeigt werden.

11. Bildanzeigeverfahren nach einem der Ansprüche 7 bis 10, wobei
das Anzeigen ein Anzeigen von Markierungsinformation, welche die Positionen der Bilder angibt, die von einer Anzeigeliste gemäß der Bildextraktionsbedingung nach der Änderung entfernt werden, an der Anzeigeeinheit (13) umfasst.

12. Bildanzeigeverfahren nach Anspruch 11, wobei
das Anzeigen ein Anzeigen der Markierungsinformation in einem Zwischenraum zwischen Bildern an der Anzeigeeinheit (13) umfasst.

13. Bildanzeigeprogramm, welches bei Ablauf auf einem Computer die Schritte des Verfahrensanspruchs 7 ausführt.

## Revendications

1. Appareil d'affichage d'images (4 ; 24 ; 34 ; 44), comprenant :
une unité d'obtention d'images (11) qui obtient une série d'images capturées selon une série chronologique ;
une unité de fixation de condition d'extraction (15c) qui fixe une condition d'extraction d'images ;
une unité d'extraction d'images (15b) qui extrait des images d'une série d'images sur la base de la condition d'extraction d'images fixée par l'unité de fixation de condition d'extraction (15c) ; et
une unité d'affichage (13) qui affiche une liste d'images extraites par l'unité d'extraction d'images (15b) ; **caractérisé en ce que**
l'unité de fixation de condition d'extraction (15c) est adaptée à changer la condition d'extraction d'images en fonction de différentes valeurs de seuil, et
l'appareil d'affichage d'images (4 ; 24 ; 34 ; 44) comprend en outre
un contrôleur d'affichage (15a ; 25a ; 35a ; 45a) qui commande l'unité d'affichage (13) de telle manière que, lorsque la condition d'extraction d'images est changée à différentes valeurs de seuil par l'unité de fixation de condition d'extraction (15c), des nouvelles images qui sont nouvellement extraites conformément à la condition d'extraction d'images post-changement, parmi des images extraites conformément à la condition d'extraction d'images qui est changée par l'unité d'extraction d'images (15b), sont affichées selon un motif d'affichage différent de celui d'images anciennes qui ont été extraites conformément à la condition d'extraction d'images pré-changement.

2. Appareil d'affichage d'images (4 ; 24 ; 34 ; 44) selon la revendication 1, dans lequel
le contrôleur d'affichage (15a ; 25a ; 35a ; 45a) commande l'unité d'affichage (13) de telle manière que les nouvelles images qui sont extraites conformément à la condition d'extraction d'images post-changement sont mises en exergue et affichées.

3. Appareil d'affichage d'images (4 ; 24 ; 44) selon la revendication 2, dans lequel
le contrôleur d'affichage (15a ; 25a ; 45a) commande l'unité d'affichage (13) de telle manière que les nouvelles images à une taille plus grande que celle des anciennes images sont mises en exergue et affichées.

4. Appareil d'affichage d'images (4 ; 34 ; 44) selon la revendication 1, dans lequel
le contrôleur d'affichage (15a ; 35a ; 45a) détermine un niveau de la condition d'extraction d'images de chaque image incluse dans les images et commande l'unité d'affichage (13) de telle manière que l'information de niveau indiquant le niveau déterminé est annexée à chaque image et ainsi les nouvelles images sont affichées selon un motif d'affichage différent de celui des images anciennes.

5. Appareil d'affichage d'images (4 ; 24 ; 34 ; 44) selon l'une quelconque des revendications 1 à 4, dans lequel
le contrôleur d'affichage (15a ; 25a ; 35a ; 45a) commande l'unité d'affichage (13) de telle manière qu'une information de marque indiquant les positions d'images qui sont supprimées d'une liste d'affichage conformément à la condition d'extraction d'images post-changement est affichée.

6. Appareil d'affichage d'images (4 ; 24 ; 34 ; 44) selon la revendication 5, dans lequel
l'unité d'affichage (13) affiche l'information de marque dans un espace entre des images affichées dans une liste.

7. Procédé d'affichage d'images, comprenant :
l'obtention d'une série d'images capturées selon une série chronologique ;
la fixation d'une condition d'extraction d'images ;
l'extraction d'images d'une série d'images sur la base de la condition d'extraction d'images ; et
l'affichage d'une liste d'images extraites ; **caractérisé par**.
le changement de la condition d'extraction d'images en fonction de différentes valeurs de seuil, et en ce que
lorsque la condition d'extraction d'images est changée à différentes valeurs de seuil, des nouvelles images qui sont nouvellement extraites conformément à la condition d'extraction d'images post-changement, parmi des images extraites conformément à la condition d'extraction d'images, sont affichées selon un motif d'affichage différent de celui d'images anciennes qui ont été extraites conformément à la condition d'extraction d'images pré-changement.

8. Procédé d'affichage d'images selon la revendication 7, dans lequel
l'affichage inclut la mise en exergue et l'affichage des nouvelles images qui sont nouvellement extraites conformément à la condition d'extraction d'images post-changement sur l'unité d'affichage (13).

9. Procédé d'affichage d'images selon la revendication 8, dans lequel
l'affichage inclut l'affichage sur l'unité d'affichage (13) des nouvelles images à une taille plus grande que celle des anciennes images de telle manière que les nouvelles images sont mises en exergue.

10. Procédé d'affichage d'images selon la revendication 7, dans lequel
l'affichage inclut la détermination d'un niveau de la condition d'extraction d'images de chaque image incluse dans les images, et la mise en annexe d'une information de niveau indiquant le niveau déterminé à chaque image de telle manière que les nouvelles images sont affichées selon un motif d'affichage différent de celui des images anciennes.

11. Procédé d'affichage d'images selon l'une quelconque des revendications 7 à 10, dans lequel
l'affichage inclut l'affichage sur l'unité d'affichage (13) d'une information de marque indiquant les positions d'images supprimées d'une liste d'affichage conformément à la condition d'extraction d'images post-changement.

12. Procédé d'affichage d'images selon la revendication 11, dans lequel
l'affichage inclut l'affichage de l'information de marque dans un espace entre les images sur l'unité d'affichage (13).

13. Programme d'affichage d'images qui exécute, lorsqu'il tourne sur un ordinateur, les étapes du procédé selon la revendication 7.
